Europäisches Patentamt

European Patent Office

Office européen des brevets

(11) Publication number: **0 253 578 B1**

# EUROPEAN PATENT SPECIFICATION

(45) Date of publication of patent specification: **21.10.92**

(51) Int. Cl.5: **G01N 33/543**, C12Q 1/00,
//G01N33/569,G01N33/76

(21) Application number: **87306087.5**

(22) Date of filing: **09.07.87**

(54) A solid phase system incorporating tetrazolium salts for use in ligand-receptor assays.

(30) Priority: **15.07.86 US 885973**

(43) Date of publication of application:
**20.01.88 Bulletin 88/03**

(45) Publication of the grant of the patent:
**21.10.92 Bulletin 92/43**

(84) Designated Contracting States:
**AT BE CH DE ES FR GB GR IT LI LU NL SE**

(56) References cited:
**EP-A- 0 200 381**
**WO-A-84/04970**
**FR-A- 2 514 511**
**US-A- 3 905 872**
**US-A- 4 351 899**

(73) Proprietor: **HYBRITECH INCORPORATED**
**11095 Torreyana Road**
**San Diego California 92126(US)**

(72) Inventor: **Anderson, Richard R.**
**634 Hollyridge Drive**
**Encinitas, CA 92023(US)**

(74) Representative: **Tapping, Kenneth George et al**
**Erl Wood Manor**
**Windlesham Surrey, GU20 6PH(GB)**

## Description

This invention relates to ligand-receptor assay processes. In another aspect, it relates to a solid phase system for use in ligand-receptor assays, particularly immunoassays using monoclonal antibodies. In another aspect it relates to a solid support system incorporating tetrazolium salts resulting in augmented color development.

Ligand-receptor assays, particularly immunoassays, provide sensitive diagnostic tools for the in vitro detection in serum and other body fluids of analytes associated with disease and other physiological conditions of clinical significance.

In the past, immunoassays typically have relied upon a polyclonal antibody preparation bound to a solid phase. In such assays, a solution of antigen, labeled to permit detection, is allowed to compete with antigen in a sample for the solid phase antibody. The extent to which the labeled antigen is bound to the solid phase or is detected in the liquid phase can be used as a measure of the presence and quantity of antigen in the sample being analyzed.

Subsequently, non-competitive immunometric assays became available. In these assays, a polyclonal antibody preparation bound to a solid phase is also used. The sample containing the suspected antigen is allowed to contact the solid phase in order for the antigen to bind to the antibodies on the solid phase. Typically, after an incubation step the sample is separated from the solid phase which is then washed and incubated with a solution of additional polyclonal antibodies which has been labeled, for example with a radionuclide, an enzyme, or a fluorescent moiety, to permit detection.

After this second incubation, the unbound labeled antibody is separated from the solid phase and the amount of labeled antibody in either the liquid phase or bound to the solid phase in an antibody:antigen:antibody sandwich is determined as a measure of the presence and/or concentration of antigen in the sample tested.

More recently, immunoassay processes have been modified to use monoclonal antibodies. For example, U.S. Patent No. 4,376,110 describes two-site immunometric assays using pairs of monoclonal antibodies, one bound to a solid phase and the other labeled to permit detection. The use of monoclonal antibody pairs which recognize different epitopic sites on an antigen has made it possible to conduct simultaneous immunometric assays in which the antigen and labeled antibody incubations do not require the intermediate steps of prior processes.

In the foregoing immunoassay processes, the solid phase system typically comprises an antibody bound to a bead, or alternatively, an antibody coated on a material such as a membrane or filter, suitable to capture an antigen of interest. Also, the solid phase system can comprise a porous matrix in which antibody coated microspheres are entrapped. Techniques for binding receptors to solid phase supports are well known in the art. For example, antibodies may be bound to polysaccharide polymers using the process described in U.S. Patent No. 3,645,090. The second antibody is labeled with an enzyme, e.g., alkaline phosphatase, which catalyzes the conversion of an added substrate, e.g., indoxyl phosphate, to a detectable colored precipitate, e.g., indigo blue. These indigogenic reactions have been found to be useful for verification of the presence of enzymes, such as alkaline phosphatases, in both the histological staining of cellular material and the colorimetric determinations of analytes in immunoassays.

In histology, for example, the formation of insoluble particulates of indigo blue are useful for precise visual imaging of cellular organelles. The insoluble particulates localize in the vicinity of the organelle possessing enzymatic activity and thus establish the presence or absence of alkaline phosphatase in specific tissue components. To achieve the best imaging, the rate of formation of the colored precipitate must be large so that the precipitate formed, in fact, is localized in the vicinity of the enzymatic activity. Unfortunately, the formation of indigo blue from indoxyl phosphate is a multistep reaction which under most reaction conditions does not follow simple first-order kinetics. This results in slower reaction times and reduced sensitivity. A kinetic improvement to a staining procedure employing indoxyl phosphate as the substrate may be achieved by including a second dye which reacts with a dephosphorylated indoxyl to form a colored precipitate other than indigo blue. Tetrazolium salts are a class of compounds which have been found to facilitate the staining of phosphatase containing organelles, subsequent to exposure to indigogenic reagents. Tetrazolium salts can react chemically with dephosphorylated indoxyl to yield the reduced form of the tetrazolium (formazan), generally an intensely colored precipitate. Since the tetrazolium salt can be added in large excess relative to the amount of transient dephosphorylated indoxyl, the rate of formation of colored precipitate more closely follows first-order kinetics. As a consequence, the degree to which the colored precipitate is localized in the vicinity of enzyme activity in cellular components is enhanced, and improved information concerning the stained sample is thus achieved.

A limitation of the two-dye technique is that reaction conditions, i.e., solutions of alkaline pH, most favorable to the detection of alkaline phosphatase are not favorable to the long-term solution phase stability of tetrazolium salts. Therefore, the mixture of indigogenic substrates and tetrazolium salts in a single alkaline solution is not practical for stability reasons. Additionally, the tetrazolum salts and the indigogenic dyes when present in the same solution are generally found to undergo slow chemical coupling even in the absence of enzyme, e.g., alkaline phosphatase. In practice these limitations require that either the solutions containing both dyes be made just prior to use, or that two separate solutions, one containing the enzyme substrate and one containing the tetrazolium salt, be used to deliver the two reactants.

The tetrazolium salt color augmentation techniques have been used also in enzymetric immunoassays to improve the kinetics and detection sensitivity in colorimetric assays which employ alkaline phosphatase as the enzyme label. These assays have also suffered from the same limitations associated with the use of tetrazolium salts in histological staining. Namely, a single solution containing both indigogenic dyes and tetrazolium salts generally do not exhibit commercially acceptable long term stability. Again, a solution to the problem is to package the enzyme substrate and tetrazolium salt in two separate solutions. An alternate solution to this problem is to significantly reduce the concentrations of either or both the enzyme substrate or tetrazolium salt when combined in a single solution containing two components. The reduced concentration retards the non-enzymatic chemical coupling of the indigogenic enzyme substrate with the tetrazolium salt and thus extends the stability of the solution containing both components. However, reduced concentration of the enzyme substrate results in reduced assay sensitivity or increased assay incubation times, neither of which is commercially desirable. Thus, any gain in assay sensitivity from pseudo-first order kinetics is offset by the reduced sensitivity resulting from the lower concentration of the enzyme substrate in the solution. As a consequence of these problems associated with the implementation of tetrazolium salts, this technique for enhancing enzymetric immunoassays has not been frequently employed, despite the substantial improvements in assay sensitivity which are potentially available.

Accordingly, a need exists for a more convenient method for employing tetrazolium salts to enhance the sensitivity of enzymetric immunoassays which use indigogenic enzymes as the enzyme label.

Generally, the present invention provides a method for incorporating tetrazolium salts in ligand-receptor assays, said assays employing an enzyme labeled receptor conjugate and indigogenic dye substrates, for the determination of at least one target ligand in a sample. The incorporation of the tetrazolium salt augments the color development of indigogenic substrates and enhances the sensitivity of the assay.

Particularly, the present invention provides a solid phase system for use in ligand-receptor assays, particularly immunoassays, for the detection of a selected analyte in a fluid sample. As used, "ligand-receptor assay" refers to an assay for an analyte which is detected by the formation of a complex between a ligand and another substance capable of specific interaction with the ligand, i.e., a receptor. The ligand may be the analyte itself or a substance which if detected can be used to infer the presence of the analyte in a sample. Persons skilled in the art will appreciate that, depending upon the analyte of interest, a member of a specific binding pair may be either receptor or ligand depending upon assay design. In the context of the present invention, the term "ligand" may encompass antigens, haptens, antibodies, deoxyribonucleic acid (DNA), ribonucleic acid (RNA), hormones, metabolites and other naturally occurring substances of diagnostic interest having a specific binding partner, i.e., the receptor of the ligand-receptor assay. In the context of the present invention, "localized" encompasses the association of ligand, receptor, or tetrazolium salt by covalent binding, noncovalent binding, chemical coupling, entrapment of coated microspheres, and adsorption by hydrophobic/hydrophobic or hydrophilic/hydrophilic interactions.

Accordingly, the solid phase system of the invention comprises a solid support on which is localized (a) a receptor capable of binding a target ligand, and (b) a tetrazolium salt capable of augmenting enzymatic color development. The solid support may be a porous matrix, bead, membrane or filter which either has the inherent characteristic of being sufficiently hydrophobic to adsorb the hydrophobic tetrazolium salt, and sufficiently hydrophilic to allow wetting by aqueous solutions or has been treated with another substance, e.g., an organic binder, that provides hydrophobic sites for the adsorption of the tetrazolium salts. The receptor may be chemically coupled, e.g., noncovalently bound, to the solid support. Alternatively, the solid support is a porous matrix in which receptor coated microspheres and tetrazolium salt coated microspheres are entrapped. Also, the solid phase system may incorporate an internal reference in which the receptor, reference receptor, and tetrazolium salt are localized on the solid support in discrete test and reference zones.

The present invention is further directed to an apparatus comprising, as a first member, a porous solid phase system as described above. The preferred apparatus further comprises, as a second member, an absorbent member associated with the solid phase system so as to facilitate the flow of a fluid sample through the solid phase system and into the second member. (The terms "solid phase system" and "first porous member" are used interchangeably.)

A particularly preferred apparatus further comprises the incorporation of an internal reference on said first porous member wherein the receptor, reference receptor, and tetrazolium salt are localized on the solid support in discrete test and reference zones.

The present invention is further directed to a ligand-receptor assay process comprising, as a first step, the introduction of a fluid sample suspected of containing a target ligand onto the first porous member of the apparatus described above. The first porous member includes a solid support on which is localized in the same area a receptor capable of binding said target ligand and a tetrazolium salt capable of augmenting indigogenic dye color development. Following addition of the sample and lapse of sufficient time for the localized receptor to bind any target ligand present, a solution of labeled receptor conjugate capable of binding with said target ligand is added to the solid phase system. The label for the receptor conjugate is an indigogenic enzyme which is capable of reacting with an indigogenic dye. After unbound receptor conjugate is washed from the solid phase system with the wash solution, a substrate containing an indigogenic dye is added. The indigogenic dye is converted by the enzyme, the product of which undergoes reaction with the localized tetrazolium salt to produce a colored precipitate. The receptor conjugate is then detected by a visual or instrumental measurement of the color development.

As used, "receptor conjugate" refers to a complex comprising a receptor and a label capable of detection. In the case of an immunometric assay for a target antigen, the receptor conjugate may be a labeled antibody, preferably a monoclonal antibody. Alternatively, if the target ligand is an antibody, labeled antigen may be used as the receptor conjugate. The presence of bound receptor conjugate on the solid phase system is an indication of the presence of the analyte in the sample.

The present invention is further directed to a method of preparing the solid phase system for use in ligand receptor assays in which the solid phase system results in augmentation of indigogenic dye color development. A preferred method comprises localising a tetrazolium salt on the solid phase support concurrently with the localization of the solid phase receptor, particularly during the entrapment of microspheres which are coated with receptor. This is accomplished by dissolving a tetrazolium salt in the fluid used to suspend the receptor-coated microspheres. The tetrazolium salt is adsorbed from the fluid onto the surface of the entrapping solid support and is available for later chemical reaction with indigogenic reagents. An especially preferred method of preparing the solid phase system of the present invention comprises adsorbing the tetrazolium salt onto the surface of synthetic microspheres and introducing these microspheres into a suspension of receptor-coated microspheres with subsequent entrapment of both types of particulates by a porous solid matrix.

This invention has been summarized in order that the detailed description that follows may be better understood.

As indicated above, the present invention provides a solid phase system for use in ligand-receptor assays, particularly immunoassays, for the detection of a selected analyte in a fluid sample.

In accordance with the present invention, the solid phase system comprises a solid support on which is localized, in the same area, a receptor and the tetrazolium salt. A variety of solid supports, including a porous matrix, bead, membrane, or filter, may be utilized in the present invention provided that they are sufficiently hydrophobic to adsorb the tetrazolium salts and sufficiently hydrophilic to allow wetting by aqueous solution. The dual hydrophobic/hydrophilic nature of the solid support may be inherent in the material used or may be imparted to the material as a result of treatment of the material, e.g., glass fibers admixed with organic binders or ceramic materials treated to provide hydrophobic binding sites. Among the materials preferred for use as a solid support are nylon, polystyrene, latex, polyethylene, and polypropylene.

The localization of the receptor and tetrazolium salt on the solid support of the solid phase system of the present invention may be accomplished in a number of ways. The receptor may be localized on the solid support by chemical coupling, e.g., covalent binding and noncovalent binding. Techniques for binding receptors to a solid support are well known in the art. See, for example, U.S. Patent No. 3,645,090, which teaches the process for binding antibodies to polysaccharide polymers. Alternatively, a particularly preferred method of localization of the receptor on the solid support in which the solid support is a porous matrix comprises entrapping receptor coated microspheres within said matrix. Methods for coating microspheres and introducing them into a porous matrix are now well-known to those skilled in the art and require no further elaboration. The tetrazolium salt may be localized on the solid support by selecting a solid support that is sufficiently hydrophobic to adsorb the hydrophobic tetrazolium salts and yet sufficiently hydrophilic to allow wetting by aqueous solutions. A particularly preferred means for localizing the tetrazolium salt comprises coating hydrophobic microspheres with the tetrazolium salt and introducing them within a porous matrix solid support where they are entrapped. The most preferred means for localizing the receptor and tetrazolium salt is by entrapment of coated microspheres.

In accordance with the present invention, the tetrazolium salts selected for use may be, for example, p-nitroblue tetrazolium chloride, m-nitro neotetrazolium chloride, and tetra-nitroblue tetrazolium chloride. All of these tetrazolium salts undergo rapid reaction with a dephosphorylated indoxyl to produce a brightly colored precipitate. Ligand receptor assays for which the present invention is useful are immunoassays, particularly immunometric assays, and nucleic acid probe-assays. The receptor of the present invention may be a nucleic acid probe sequence for the target ligand, or an antigen or antibody capable of capturing a target ligand. A particularly preferred antibody is a monoclonal antibody.

The present invention is also useful if the solid phase system provides a method of incorporating an internal reference which allows quantitative determination of ligand concentrations in sample or qualitative confirmation of proper assay protocol. The solid phase system of the present invention incorporating an internal reference would include a solid support comprising at least one discrete test zone and at least one discrete reference zone on which are localized said receptor and said tetrazolium salt, and a reference receptor and a tetrazolium salt, respectively. The method for incorporating an internal reference in a analyte-receptor assay is the subject matter of pending European Application No. 87302403.8, filed March 20, 1987, The present invention is capable of providing augmented color development at both the test and reference zones. Also, a particular advantage to the use of tetrazolium salts to augment the color development is that the resulting increased sensitivity of the assay diminishes the need to select a reference receptor so the rate constants for the binding of the receptor conjugate to the reference receptor and to the target ligand are substantially equivalent.

Generally, when preparing the solid phase system of the present invention one must consider the following factors. A solution of a tetrazolium salt (an organic compound of limited aqueous solubility, i.e., a somewhat hydrophobic compound) is applied to a synthetic solid phase support which exhibits surface characteristics of both a hydrophilic and hydrophobic nature. Since tetrazolium salts possess significant hydrophobic characteristics, they are readily adsorbed to surfaces which exhibit hydrophobic characteristics such as possessed by many synthetic polymeric materials, e.g., porous nylon membrane or latex microspheres. The adhesion of a tetrazolium salt to a synthetic polymer via hydrophobic interactions is of sufficient strength that the tetrazolium salt is not readily removed by exposure to substantial volumes of aqueous solution. Nevertheless, the tetrazolium salt retains sufficient chemical accessibility such that it remains capable of chemical reaction with appropriate reagents, such as the reaction products from the enzymatic dephosphorylation by alkaline phosphates of indoxyl phosphate.

Accordingly, a method for the introduction of the tetrazolium salt onto the surface of the solid support is to dissolve the tetrazolium salt in the neutral pH solution employed by a heterogeneous enzymetric immunoassay to effect the separation between solution phase and solid phase immobilized receptor conjugate (i.e., free-bound separation). This method of introduction of the tetrazolium salts has the advantage that a separate solution, whose only function is for introduction of the tetrazolium salt, is not required. The indigogenic reagents used as enzymes substrates are generally introduced as solutions whose pH is substantially alkaline since maximal enzyme activity is achieved at alkaline pH. Alkaline solutions of tetrazolium salts, however, are generally found to be unstable, because alkaline pH conditions promote spontaneous reduction of the tetrazolium salt to the reduced form of the dye (i.e., the formazan form). The use of a neutral pH solution substantially obviates this limitation. The subsequent chemical reaction between enzymatically cleaved indigogenic alkaline phosphatase substrates and the tetrazolium salt adsorbed onto the solid phase support is facile.

The present invention further provides a method for preparing a solid phase system which provides a means for augmenting indigogenic dye color development. One method for preparing the solid phase system comprises localization of the tetrazolium salt by dissolution of the tetrazolium salt in the aqueous fluid used to form the receptor-coated microsphere suspension prior to introduction onto a porous solid support. The porous solid support must possess both hydrophobic and hydrophilic surface characteristics. The tetrazolium salt will partition between the aqueous suspension fluid and the hydrophobic regions of the porous solid support. With a carefully prepared porous solid support a substantial proportion of the dissolved tetrazolium salt will adhere to the porous support and will be localized exclusively in the vicinity of the entrapped microspheres. The aqueous suspension fluid is removed by evaporation leaving behind both the adsorbed tetrazolium salt and the entrapped protein-coated microspheres. As noted before, the hydrophobic binding forces between the tetrazolium salt and the solid phase support are of sufficient strength so that subsequent exposure of the tetrazolium salt treated support to aqueous solutions commonly used in the performance of immunoenzymetric assays will not lead to significant leaching of the tetrazolium salt from the surface of the porous solid phase support. The tetrazolium salt adsorbed to the porous solid phase support via this method remains capable of facile reaction with the products of enzymatic cleavage of indigogenic dyes. Problems associated with the solution phase stability of tetrazolium salts are avoided by the present invention since the tetrazolium is stored in the dry solid phase system until rehydrated during the ligand receptor immunoenzymetric assay.

Particularly preferred as a method for localization of the tetrazolium salt is coating tetrazolium salt onto synthetic polymeric microspheres. In this method the tetrazolium salt is adsorbed to untreated microspheres via hydrophobic interactions between the tetrazolium dye and the polymeric microspheres. Microspheres prepared in the presence of tetrazolium salts are mixed with microspheres coated with receptor such as monoclonal antibodies and a uniformly commingled suspension of these two types of coated microspheres is applied to a porous matrix solid support, the particulates being entrapped by the porous support. As related previously, the tetrazolium salts present on the appropriately coated microspheres are available for reaction with the enzymatically cleaved products of indigogenic substrates. In this method of the present invention the tetrazolium salt is subject to the same advantages available as when the tetrazolium salt is dissolved in the fluid in which the receptor-coated microspheres are suspended. An additional advantage of using tetrazolium salt-coated microspheres is that the hydrophobic/hydrophilic characteristics of the adsorbing medium are defined by the surface characteristics of the microspheres. As such, these characteristics are independent of the surface characteristics of the porous solid phase support, permitting the restrictions in the selection of the porous solid support to be relaxed.

Also preferred for use in the present invention is the following method for localization of tetrazolium salt onto the solid phase support. Antibody against a selected antigen is coupled either convalently or noncovalently to the solid phase support in a selected reaction zone. A volume of an aqueous solution containing a tetrazolium salt is dispensed onto the solid phase support in the region used to localize the antibody. After evaporative removal of the bulk fluid, the solid phase support is used to perform the assay. The adsorbed tetrazolium salt is available as indicated above for chemical reaction with the enzymatically cleaved indigogenic reagents. This method of the invention exhibits the same advantages already listed for methods in which the tetrazolium is applied prior to the immunoassay and is stored in the dry phase in the intervening time between application to the solid phase and use in an immunoassay.

Also useful for the present invention is the following method for introduction of a tetrazolium salt onto the solid phase support. Antibody against a selected antigen is coupled noncovalently to latex microspheres and then a volume of the antibody-coated microspheres is localized on the solid phase support in a selected reaction zone. A volume of an aqueous solution containing a tetrazolium salt is dispensed onto the solid phase support in the region used to localize the microspheres. As before, after evaporative removal of the bulk fluid, the prepared solid phase support is used to perform an immunoenzymetric assay. The adsorbed tetrazolium is available for chemical reaction with the enzymatically cleaved indigogenic substrate. This method of the invention also exhibits the advantages listed for methods in which the tetrazolium is applied prior to the immunoassay and is stored in the dry phase in the time intervening between application to the solid phase and application in an immunoassay.

The apparatus of the present invention comprises, as a first porous member, a solid phase system as described above. The apparatus further comprises additional means, operatively associated with the first porous member, for facilitating the flow of a fluid sample and liquid reagents through the first member. Among the means which may be suitably utilized are means for applying a vacuum source or capillary action below the porous member to draw liquid through the porous member. Alternatively, the additional means may comprise means for applying a positive pressure above the porous member to force the sample liquid or reagents through that member. In a particularly preferred embodiment, a second absorbent member is associated with the solid phase system so as to permit the flow of a fluid sample through the solid phase system and into the absorbent material. The second absorbent member, having a surface over which the solid phase may be placed, has capillaries therethrough in a direction generally transverse to the surface over which the solid phase system is placed such that the capillaries are in communication with the pores of the solid phase system. A variety of materials may be used for the absorbent member, such as cellulose acetate fibers or polyester.

In a particularly preferred embodiment, the first porous member further comprises a solid support with at least one discrete test zone and at least one discrete reference zone on which are localized said receptor and said tetrazolium salt, and a reference receptor and said tetrazolium salt, respectively. The test and reference zones provide a means for quantitative determination of analyte concentrations in sample or qualitative confirmation of proper assay protocols substantially independent of normal variations in assay conditions.

As previously indicated, the solid phase system and apparatus of the present invention are of significant utility in the performance of ligand-receptor assays, particularly multiple ligand-receptor assays for the detection of at least two analytes of interest and/or ligand-receptor assays incorporating an internal control system. While the present invention is particularly useful for the performance of immunoassays, those skilled in the art will appreciate that with suitable modification, the solid phase system provided by the invention may be utilized for other ligand-receptor assays, including assays involving nucleic acid probe technology.

In accordance with the ligand-receptor assay processes of the present invention, a fluid sample suspected of containing the analyte(s) of interest is introduced onto the solid phase system of an apparatus as described above. In the case of an immunoassay, the solid phase system comprises a solid support on which is localized in the same area a receptor and a tetrazolium salt. Following the flow of fluid sample through the solid phase system and into the absorbent member, a solution of receptor conjugate, capable of binding with the target ligand and labeled with an indigogenic enzyme so as to permit detection, is added. In the case of immunoassays, the receptor conjugate may be an antibody, preferably a monoclonal antibody, or antigen capable of binding with the ligand of interest. The addition of receptor conjugate, as appropriate, permits the formation of a complex with the ligand captured by the solid phase system. In the case of an immunoassay, if the solid support comprising the solid phase system is localized with monoclonal antibody, and the enzyme labeled receptor conjugate is also a monoclonal antibody, the two antibodies are selected to bind to non-interfering binding sites of the antigen, essentially as described in U.S. Patent Nos. 4,376,110 and U.S. 4,486,530. In the presently preferred embodiments, the receptor conjugate is labeled with an indigogenic enzyme. After the solution of receptor conjugate has flowed through the solid phase system, a washing solution may be added to remove unbound receptor conjugate. Thereafter, the receptor conjugate complexed with the ligand of interest is detected. If an enzyme has been selected as the label component, the bound receptor is determined by the addition of an indigogenic enzyme substrate to the solid phase system. Upon reaction with the substrate, the enzyme will generate, if properly selected, a dephosphorylated indoxyl which will react with the localized tetrazolium salt to generate a brightly colored precipitate.

In the process of the present invention the indigogenic dye substrate and indigogenic enzyme pairs suitable for use include indoxyl phosphate/alkaline phosphatase, indoxyl glucoside/alkaline glucosidase, indoxyl sulfate/alkaline sulfatase and indoxyl acetate/alkaline esterase. The process of the present invention is useful in ligand receptor assays that include immunoassays, particularly immunometric assays, and nucleic acid probe assays. Receptors useful in the processes of the present invention include antibodies, antigens, allergens, and nucleic acid sequences complimentary to portions of the nucleic acid sequence of a target ligand. Preferably the receptor is a monoclonal antibody and the receptor conjugate is a labeled monoclonal antibody which are selected to bind at non-interfering epitopes on the ligand.

A preferred material for the microspheres used in the present invention is latex.

It will be recognized by those skilled in the art that assays for a broad range of analytes may be performed in accordance with the present invention. Potential analytes may include, for example, antigens such as human chorionicgonadotropin, prostatic acid phosphatase, prostate-specific antigen, alphafetoprotein, carcinoembryonic antigen, luteinizing hormone, creatine kinase isoenzymes and other antigens in serum, plasma, urine or other biological fluids. Additionally, the present invention is useful for the assay of viruses, bacteria, parasites or fungi, or antigens or antibodies associated therewith, including, for example, Rubella virus, Rota virus, adenovirus, respiratory syncytial virus, HTLV, hepatitis virus, hepatitis/A, hepatitis/B, hepatitis nonA nonB, influenza virus, cytomegalovirus and herpes virus, as well as group A and group B streptococcus, Neisseria gonorrhea, Trichomonas vaginalis, Candida albicans, Chlamydia trachomatis and Hemophilus influenza.

Further, those skilled in the art having the benefit of this disclosure will appreciate that the present invention is applicable to assays involving nucleic acid probe technology. Specifically, a nucleic acid sequence complementary to a portion of the nucleic acid sequence of a ligand of interest may be bound to microspheres which are thereafter entrapped in a porous matrix. Such a solid phase system may be incorporated in an apparatus as previously described and utilized in assay processes. To perform such assay processes, a fluid sample suspected of containing the target ligand is introduced onto the solid phase and the ligand of interest is captured on the solid phase. Thereafter, a labeled nucleic acid probe having a nucleic acid sequence complementary to a different portion of the nucleic acid sequence of the target ligand is added to permit the formation of a complex of the labeled nucleic acid probe and the ligand captured on the solid phase. The detection of the labeled nucleic acid probe bound to the solid phase system provides an indication of the presence of the analyte in a given sample.

The following examples are provided to further illustrate the invention:

EXAMPLE I

BINDING OF TETRAZOLIUM SALT TO POROUS SOLID PHASE SUPPORT VIA MICROSPHERE SUSPENSION CARRIER SOLUTION

Microspheres were coated with a monoclonal antibody against HCG by the method of passive adsorption of protein to a latex surface. The antibody coated microspheres were then suspended in a solution containing 10 mM sodium phosphate, 150 mM sodium chloride, 0.1% by weight sodium azide, 10% by weight sucrose and approximately 1 g/L p-nitro-blue tetrazolium chloride whose pH was 7.0. A volume of antibody coated microspheres and the tetrazolium containing suspension solution was deposited onto a porous nylon membrane and the membrane allowed to dry. An immunoassay using an ICON device containing the treated membrane was then performed for serum HCG using 0.250 mL of HCG containing serum followed by 0.150 mL of an anti-HCG antibody: alkaline phosphatase receptor:enzyme conjugate. The receptor:enzyme conjugate was allowed to incubate with the immobilized antibody:HCG complex for 1 minute and then a bound/unbound separation was achieved with the addition of 1 mL of an aqueous wash solution. 0.150 mL of a solution containing indoxyl phosphate was allowed to react with the immobilized alkaline phosphatase for 1 minute. In the absence of p-nitro-blue tetrazolium, the developed reaction is a blue characteristic of indigo blue. On the membrane which contained both entrapped microspheres against HCG and adsorbed tetrazolium the developed color was purplish which is characteristic of the formazan form of p-nitro-blue tetrazolium chloride.

EXAMPLE II

BINDING OF TETRAZOLIUM SALT TO LATEX MICROSPHERES PRIOR TO ENTRAPMENT ON A POROUS SOLID SUPPORT

A solution whose composition was 10 mM sodium phosphate, 150 mM sodium chloride, 0.1% by weight sodium azide, 10% by weight sucrose, and approximately 1 g/L p-nitro-blue tetrazolium chloride at a solution pH of 7.0 was used to suspend uncoated latex microspheres to a concentration of approximately 1% by weight. The microspheres were allowed to incubate overnight at room temperature with occasional gentle agitation. The microspheres suspension was centrifuged to pellet the microspheres and the tetrazolium containing solution was decanted away. The tetrazolium-coated microspheres were resuspended in a solution of composition 10 mM sodium phosphate, 150 mM sodium chloride, 0.1% by weight sodium azide and 10% by weight sucrose at a pH of 7.0 to a concentration of 1% by weight latex microspheres. The resuspended tetrazolium coated-microspheres were then mixed in a 1:1 ratio with latex microspheres coated with a monoclonal antibody against HCG. A volume of the resulting mixture of tetrazolium-coated microspheres and antibody-coated microspheres was deposited on a porous nylon membrane and allowed to air-dry. The microsphere containing membrane was inserted into an ICON™ device and an immunoassay for HCG was performed as follows: 0.250 mL of HCG-containing sample was added followed by 0.150 mL of anti-HCG antibody:alkaline phosphatase receptor:enzyme conjugate and allowed to incubate for 1 minute. A bound/unbound receptor:enzyme conjugate separation was achieved by adding approximately 1 mL of a wash solution. After allowing the wash solution to drain, 0.150 mL of an enzyme substrate solution containing indoxyl phosphate was added and allowed to undergo enzyme substrate turnover for 1 minute. The resulting reaction zone was purplish in color showing the presence of the formazan form of p-nitro-blue tetrazolium chloride and not indigo blue as would be expected from the simple reaction product of indoxyl phosphate and alkaline phosphatase.

**Claims**

1. A solid-phase system for use in a ligand-receptor assay for the detection of a selected analyte in a fluid sample comprising a solid support on which is localized in the same area:
   a) a receptor capable of binding a target ligand; and
   b) a tetrazolium salt capable of augmenting indigogenic enzymatic color development.

2. A solid phase system as claimed in Claim 1 in which the solid support is a porous matrix, bead, membrane or filter.

3. A solid phase system as claimed in Claim 2 in which the porous matrix, bead, membrane or filter is a material selected from nylon, polystyrene, latex, polyethylene, polypropylene, polystyrene, glass fibers admixed with organic binders, or ceramic materials treated to provide hydrophobic binding sites.

4. A solid phase system as claimed in Claim 1 in which the solid support is a porous matrix in which receptor coated microspheres are localized by entrapment.

5. A solid phase system as claimed in Claim 1 in which the solid support is a porous matrix in which tetrazolium salt coated microspheres are localized by entrapment.

6. A solid phase system as claimed in Claim 1 in which the solid support is a porous matrix in which both receptor coated microspheres and the tetrazolium salt coated microspheres are localized by entrapment.

7. A solid phase system as claimed in Claim 5 in which the receptor is localized on the solid support by chemical coupling.

8. A solid phase system as claimed in Claim 4 in which the tetrazolium salt is localized on the solid support by adsorption due to hydrophobic interactions.

9. A solid phase system as claimed in any one of Claims 1 to 8 in which the tetrazolium salt is p-nitroblue tetrazolium chloride, m-nitro neotetrazolium chloride, or tetra nitroblue tetrazolium chloride.

10. A solid phase system as claimed in Claim 1 in which the ligand-receptor assay is an immunoassay.

11. A solid phase system as claimed in Claim 1 in which the ligand-receptor assay is a nucleic acid probe assay.

12. A solid phase system as claimed in Claim 1 in which the receptor is an antibody or antigen capable of capturing the target ligand.

13. A solid phase system as claimed in Claim 12 in which the antibody is a monoclonal antibody.

14. A solid phase system of Claim 1 in which the solid support further comprises at least one discrete test zone and at least one discrete reference zone on which are localized said receptor and said tetrazolium salt, and a reference receptor and said tetrazolium salt, respectively.

15. An apparatus for use in a ligand-receptor assay for the detection of at least one selected analyte in a fluid sample comprising:
    a) a first porous member comprising a solid phase system according to any one of Claims 1 to 14; and
    b) a means, operatively associated with said first porous member, for facilitating the flow of said fluid sample and liquid reagents used in said assay through said first porous member.

16. An apparatus as claimed in Claim 15 in which the flow facilitating means comprises an absorbent member associated with said first member so as to permit the flow of said fluid sample and liquid reagents used in said assay through said first member and to said absorbent member, said absorbent member having a surface over which said first member is placed and having capillaries therethrough in a direction generally transverse to the surface over which said first member is placed, which capillaries are in communication with the pores of the said first member so as to draw fluid which has permeated said first member into the capillaries of said absorbent member.

**Patentansprüche**

1. Festphasensystem zur Verwendung in einem Liganden-Rezeptor-Assay für den Nachweis eines ausgewählten Analyten in einer Probeflüssigkeit, umfassend einen festen Träger, auf dem im gleichen Bereich lokalisiert sind:
    a) ein Rezeptor, der mit einem Zielliganden binden kann, und
    b) ein Tetrazoliumsalz, das die indigogene enzymatische Farbentwicklung verstärken kann.

2. Festphasensystem nach Anspruch 1, worin der feste Träger eine poröse Matrix, ein Kügelchen, eine Membran oder ein Filter ist.

3. Festphasensystem nach Anspruch 2, worin das Material für die poröse Matrix, das Kügelchen, die Membran oder der Filter ausgewählt ist aus Nylon, Polystyrol, Latex, Polyethylen, Polypropylen, Polystyrol, Glasfasern gemischt mit organischen Bindemitteln oder keramische Materialien, die behandelt wurden, um hydrophobe Bindungsstellen zu schaffen.

4. Festphasensystem nach Anspruch 1, worin der feste Träger eine poröse Matrix ist, worin mit Rezeptor beschichtete Mikrokügelchen durch Einschluß lokalisiert sind.

5. Festphasensystem nach Anspruch 1, worin der feste Träger eine poröse Matrix ist, worin mit Tetrazoliumsalz beschichtete Mikrokügelchen durch Einschluß lokalisiert sind.

6. Festphasensystem nach Anspruch 1, worin der feste Träger eine poröse Matrix ist, worin sowohl rezeptorbeschichtete Mikrokügelchen als auch tetrazoliumsalzbeschichtete Mikrokügelchen durch Einschluß lokalisiert sind.

7. Festphasensystem nach Anspruch 5, worin der Rezeptor auf dem festen Träger durch chemische Kupplung lokalisiert ist.

8. Festphasensystem nach Anspruch 4, worin das Tetrazoliumsalz auf dem festen Träger durch Adsorption aufgrund hydrophober Wechselwirkungen lokalisiert ist.

9. Festphasensystem nach einem der Ansprüche 1 bis 8, worin das Tetrazoliumsalz p-Nitroblautetrazoliumchlorid, m-Nitroneotetrazoliumchlorid oder Tetranitroblautetrazoliumchlorid ist.

10. Festphasensystem nach Anspruch 1, worin der Liganden-Rezeptor-Assay ein Immunoassay ist.

11. Festphasensystem nach Anspruch 1, worin der Liganden-Rezeptor-Assay ein Nukleinsäuresonden-Assay ist.

12. Festphasensystem nach Anspruch 1, worin der Rezeptor ein Antikörper oder ein Antigen ist, das fähig ist, den Zielliganden einzufangen.

13. Festphasensystem nach Anspruch 12, worin der Antikörper ein monoklonaler Antikörper ist.

14. Festphasensystem nach Anspruch 1, worin der feste Träger weiterhin mindestens eine diskrete Testzone und mindestens eine diskrete Referenzzone umfaßt, auf denen Rezeptor und Tetrazoliumsalz bzw. Referenzrezeptor und Tetrazoliumsalz lokalisiert sind.

15. Vorrichtung zur Verwendung in einem Liganden-Rezeptor-Assay für den Nachweis von mindestens einem ausgewählten Analyten in einer Probeflüssigkeit umfassend:
a) ein erstes poröses Glied umfassend ein Festphasensystem nach einem der Ansprüch 1 bis 14 und

b) ein Mittel, das mit dem ersten porösen Glied zusammenwirkt, um das Fließen der Probeflüssigkeit und der flüssigen Reagenzien, die in dem Test verwendet werden, durch das erste poröse Glied zu erleichtern.

16. Vorrichtung nach Anspruch 15, worin die das Fließen erleichternde Einrichtung ein absorbierendes Glied umfaßt, das mit dem ersten Glied in Verbindung steht, so daß das Fließen der Probeflüssigkeit und der flüssigen Reagenzien, die in dem Test verwendet werden, durch das erste Glied und zu dem absorbierenden Glied möglich wird, wobei das absorbierende Glied eine Oberfläche hat, oberhalb der das erste Glied angeordnet ist, und Kapillaren aufweist in einer Richtung, die im wesentlichen diagonal ist zur Oberfläche, oberhalb der das erste Glied angeordnet ist, wobei die Kapillaren in Verbindung stehen mit den Poren des ersten Glieds, um die Flüssigkeit, die durch das erste Glied gewandert ist, in die Kapillaren des absorbierenden Gliedes zu ziehen.

## Revendications

1. Système en phase solide destiné à être utilisé dans un dosage faisant appel à un ligand-récepteur pour la détection d'un analyte sélectionné dans un échantillon de fluide, comprenant un support solide sur lequel on a mis en place dans la même zone :
a) un récepteur capable de se lier à un ligand-cible; et
b) un sel de tétrazolium capable d'augmenter le développement de la couleur enzymatique indigogène.

2. Système en phase solide selon la revendication 1, dans lequel le support solide est une matrice poreuse, une bille, une membrane ou un filtre.

3. Système en phase solide selon la revendication 2, dans lequel la matrice poreuse, la bille, la membrane ou le filtre est une matière choisie parmi le nylon, le polystyrène, le latex, le polyéthylène, le polypropylène, des fibres de verre mélangées à des liants organiques, ou encore des matières céramiques traitées pour procurer des sites de liaison hydrophobes.

4. Système en phase solide selon la revendication 1, dans lequel le support solide est une matrice poreuse dans laquelle on met en place, en les retenant dans un piège, des microsphères enrobées à l'aide du récepteur.

**5.** Système en phase solide selon la revendication 1, dans lequel le support solide est une matrice poreuse dans laquelle on met en place, en les retenant dans un piège, des microsphères enrobées à l'aide de sel de tétrazolium.

**6.** Système en phase solide selon la revendication 1, dans lequel le support solide est une matrice poreuse dans laquelle on met en place, en les retenant dans un piège, à la fois des microsphères enrobées à l'aide du récepteur et les microsphères enrobées à l'aide du sel de tétrazolium.

**7.** Système en phase solide selon la revendication 5, dans lequel on met en place le récepteur sur le support solide par couplage chimique.

**8.** Système en phase solide selon la revendication 4, dans lequel on met en place le sel de tétrazolium sur le support solide par adsorption due à des interactions hydrophobes.

**9.** Système en phase solide selon l'une quelconque des revendications 1 à 8, dans lequel le sel de tétrazolium est le chlorure de p-nitro-bleu de tétrazolium, le chlorure de m-nitro-néotétrazolium ou le chlorure de tétranitro-bleu de tétrazolium.

**10.** Système en phase solide selon la revendication 1, dans lequel le dosage faisant appel à un ligand-récepteur est un immunodosage.

**11.** Système en phase solide selon la revendication 1, dans lequel le dosage faisant appel à un ligand-récepteur est un dosage faisant appel à une sonde d'acides nucléiques.

**12.** Système en phase solide selon la revendication 1, dans lequel le récepteur est un anticorps ou un antigène capable de capturer le ligand-cible.

**13.** Système en phase solide selon la revendication 12, dans lequel l'anticorps est un anticorps monoclonal.

**14.** Système en phase solide selon la revendication 1, dans lequel le support solide comprend, en outre, au moins une zone discrète d'essai et au moins une zone discrète de référence sur lesquelles sont mis en place ledit récepteur et ledit sel de tétrazolium, ainsi qu'un récepteur de référence et ledit sel de tétrazolium, respectivement.

**15.** Appareil destiné à être utilisé dans un dosage faisant appel à un ligand-récepteur pour la détection d'au moins un analyte sélectionné dans un échantillon de fluide comprenant :

a) un premier élément poreux comprenant un système en phase solide selon l'une quelconque des revendications 1 à 14; et

b) un moyen associé, lors de la mise en oeuvre, avec ledit premier élément poreux pour faciliter l'écoulement dudit échantillon de fluide et des réactifs liquides utilisés dans ledit dosage à travers ledit premier élément poreux.

**16.** Appareil selon la revendication 15, dans lequel le moyen facilitant l'écoulement comprend un élément absorbant associé audit premier élément de façon à permettre l'écoulement dudit échantillon de fluide et des réactifs liquides utilisés dans ledit dosage à travers ledit premier élément et en direction dudit élément absorbant, ledit élément absorbant étant muni d'une surface par-dessus laquelle on place ledit premier élément et étant muni de capillaires qui le traversent dans une direction généralement transversale à la surface par-dessus laquelle on place ledit premier élément, lesdits capillaires se trouvant en communication avec les pores dudit premier élément de façon à tirer le fluide qui a pénétré à travers le premier élément, dans les capillaires dudit élément absorbant.